# EUROPÄISCHE PATENTSCHRIFT

(11) **EP 2 344 061 B1**
(45) Veröffentlichungstag und Bekanntmachung des Hinweises auf die Patenterteilung: **23.07.2014**
(21) Anmeldenummer: 09777891.4
(22) Anmeldetag: 14.08.2009
(51) Int. Cl.: A61B 18/12

(54) **ELEKTROCHIRURGISCHER HF-GENERATOR**
ELECTROSURGICAL HF GENERATOR
GÉNÉRATEUR ÉLECTROCHIRURGICAL HAUTE FRÉQUENCE

(30) Priorität: 27.08.2008 DE 102008039884
(43) Veröffentlichungstag der Anmeldung: 20.07.2011
(73) Patentinhaber: ERBE Elektromedizin GmbH, 72072 Tübingen (DE)
(72) Erfinder: WERNER, Erich, 72872 Wannweil (DE)
(74) Vertreter: Bohnenberger, Johannes
(86) Internationale Anmeldenummer: PCT/EP2009/005916
(87) Internationale Veröffentlichungsnummer: WO 2010/025818

(56) Entgegenhaltungen:
- EP-A- 1 532 952
- EP-A- 1 693 015
- EP-A- 1 810 629
- EP-A- 1 836 985
- WO-A-2004/062516
- DE-A1- 3 904 558
- DE-A1- 10 046 592
- GB-A- 2 214 430

## Beschreibung

Die Erfindung betrifft einen elektrochirurgischen HF-Generator nach dem Oberbegriff des Patentanspruches 1.

In der modernen Chirurgie werden häufig elektrochirurgische HF-Generatoren zum Schneiden oder Koagulieren von biologischem Gewebe eingesetzt. Dazu wird ein HF-Signal mit einer Frequenz größer 300 kHz durch einen elektrochirurgischen HF-Generator erzeugt und von einem Operateur über eine Elektrode der zu behandelnden Stelle zugeführt. Elektrochirurgische HF-Generatoren umfassen ein Netzgerät zur Speisung einer Generatorstufe, die eine HF-Schwingung in einem Ausgangsfilter aus einem Parallelschwingkreis und einem Serienschwingkreis anregt. Ein solcher elektrochirurgischer HF-Generator ist beispielsweise aus der DE 39 04 558 A1 bekannt.

Ein weiterer Generator, der die Merkmale im Oberbegriff von Anspruch 1 aufweist, ist aus der EP 1 693 015 A2 bekannt.

Der Erfindung liegt die Aufgabe zugrunde, einen elektrochirurgischen HF-Generator zum Schneiden der Koagulieren von biologischem Gewebe mit einer verbesserten Kurzschlussfestigkeit zu schaffen.

Diese Aufgabe ist durch einen elektrochirurgischen HF-Generator nach Anspruch 1 gelöst. Vorteilhafte Ausführungsformen sind in den abhängigen Ansprüchen angegeben.

Der elektrochirurgische HF-Generator hat ein Netzgerät zum Speisen einer Generatorstufe, welche eine HF-Schwingung in einem Ausgangsfilter aus einem Parallelschwingkreis und einem damit gekoppelten Serienschwingkreis erzeugt. Der Serienschwingkreis hat eine Resonanzfrequenz, die über der Resonanzfrequenz des Parallelschwingkreises liegt.

Die Resonanzfrequenz des Serienschwingkreises ist dabei kleiner als die sich bei einem Kurzschluss des elektrochirurgischen HF-Generators einstellende Frequenz der von dem elektrochirurgischen HF-Generator erzeugten HF-Schwingung. Vorzugsweise ist die Resonanzfrequenz des Serienschwingkreises kleiner als das 0,9-fache, besonders bevorzugt kleiner als das 0,8-fache, der sich bei einem Kurzschluss des elektrochirurgischen HF-Generators einstellenden Frequenz des HF-Signals. Dadurch kann ein kurzschlussfester Ausgangsfilter erreicht werden.

Vorzugsweise ist die Resonanzfrequenz des Serienschwingkreises zwischen 5% und 25%, besonders bevorzugt zwischen 7,5% und 12%, über der Resonanzfrequenz des Parallelschwingkreises. Überraschenderweise zeigt es sich, dass bei dieser Wahl der Resonanzfrequenzen ein besonders hoher Wirkungsgrad unter einer typischen Last erreicht wird.

Vorzugsweise ist der Parallelschwingkreis über einen Übertrager mit dem Serienschwingkreis gekoppelt. Dadurch sind die beiden Schwingkreise galvanisch voneinander getrennt. Entsprechend ist ein mit dem elektrochirurgischen HF-Generator behandelter Patient galvanisch vom Netzgerät getrennt.

Ein besonders einfacher Aufbau des Ausgangsfilters ergibt sich, wenn die Induktivität des Parallelschwingkreises eine Induktivität des Übertragers, z.B. dessen Primärwicklung, ist.

Vorzugsweise hat der Serienschwingkreis beidseits des Übertragers, d.h. beidseits dessen Sekundärwicklung je eine Induktivität, denen je eine Kapazität nachgeschaltet ist, welche wiederum jeweils mit einer Elektrode verbunden sind.

Es ist vorteilhaft, wenn die Generatorstufe eine Schaltstufe zum Anregen der Schwingung in dem Ausgangsfilter umfasst, weil die Anregungsfrequenz einer Schaltstufe einfach zu regeln ist.

Zur Erzeugung eines an die Frequenz des elektrochirurgischen HF-Generators angepassten Ansteuersignals kann die Generatorstufe einen Steuereingang haben, der über einen Kondensator an den Parallelschwingkreis gekoppelt ist. Das über den Kondensator aus dem Parallelschwingkreis ausgekoppelte Signal kann einem Phasenschieber zugeführt werden, dem eine Pulsformerstufe nachgeschaltet ist.

Über einen von einem Operateur zu betätigenden Schalter, z.B. einen Hand oder Fußtaster, kann das Signal der Pulsformerstufe zu einer Treiberstufe geführt werden.

Anhand der Zeichnungen wird die Erfindung schematisch vereinfacht beschrieben. Es zeigen:
- Fig. 1: Ein Prinzipschaltbild eines elektrochirurgischen HF-Generators und
- Fig. 2: die Generator-Eingangsimpedanz und die Serienkreisimpedanz des elektrochirurgischen HF-Generators als Funktion der Frequenz des HF-Signals.

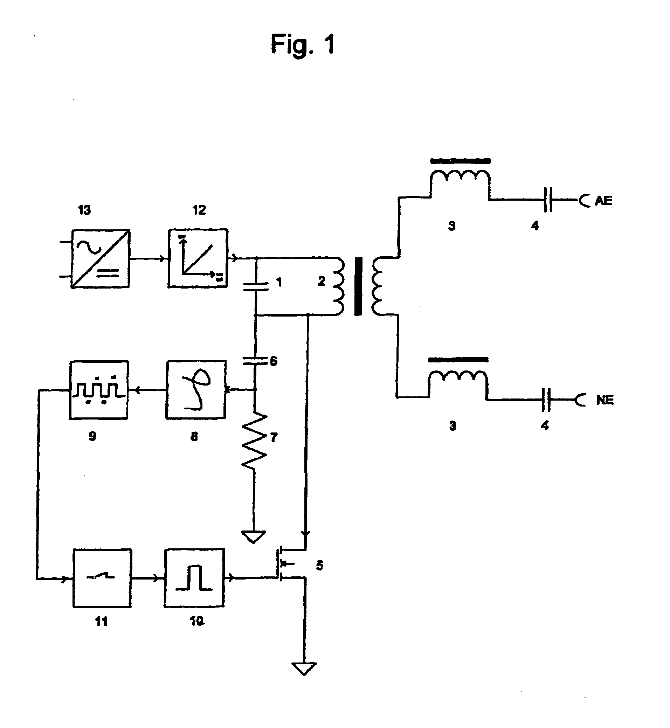

Der elektrochirurgische HF-Generator in Fig. 1 wird über eine Netzeingangsstufe 13 versorgt. Diese speist ein geregeltes Netzteil 12, das mit einem Parallelschwingkreis aus einer Kapazität 1 und einer Induktivität 2 verbunden ist. Die Induktivität 2 ist die Primärwicklung eines Übertragers. Der Übertrager koppelt den Parallelschwingkreis 1,2 mit einem Serienschwingkreis aus je einer Serienkreisdrossel 3 beidseits der Sekundärwicklung des Übertragers, denen je ein Auskoppelkondensator 4 nachgeschaltet ist. An die Auskoppelkondensatoren 4 ist je eine Elektrode angeschlossen. Der Parallelschwingkreis 1, 2 und der Serienschwingkreis 3, 4 bilden einen Ausgangsfilter. Die zur Ansteuerung des Ausgangsfilters benötigten Impulse werden über einen Kondensator 6 und einen Widerstand 7 aus dem Parallelschwingkreis 1, 2 ausgekoppelt. Zwischen dem Kondensator 6 und dem Widerstand 7 wird das Signal des Parallelschwingkreises 1,2 abgegriffen und einem Phasenschieber 8 zugeführt. Dem Phasenschieber 8 ist eine Pulsformerstufe 9 nachgeschaltet. Phasenschieber 8 und Pulsformerstufe 9 erzeugen ein auf die vorliegende Schwingung des Ausgangsfilters synchronisiertes Ansteuersignal, das über ein Bedienteil 11 einer Treiberstufe 10 zugeführt wird. Das Bedienteil 11 kann z.B. ein Hand- oder Fußtaster sein und wird von einem Operateur zur Betätigung des elektrochirurgischen HF-Generators bedient. Die Treiberstufe 10 steuert einen als Mosfet ausgeführten Schalter 5, welcher den Parallelschwingkreis 1, 2 mit dem geregelten Netzteil 12 verbindet.

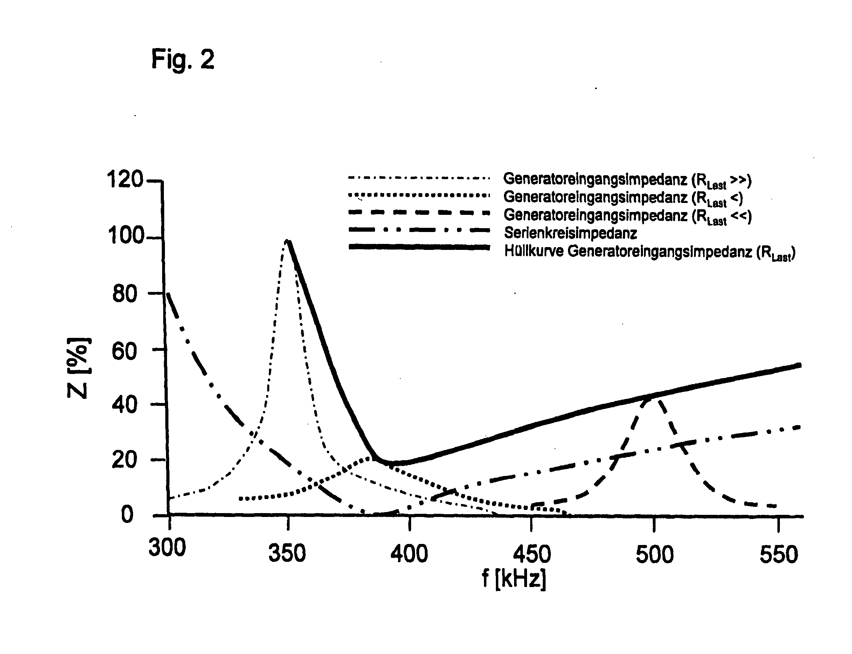

Die Serienkreisdrosseln 3 und die Auskoppelkondensatoren 4 sind so bemessen, dass die Resonanzfrequenz des Serienschwingkreises 3, 4 über der Resonanzfrequenz des Parallelschwingkreises 1, 2 liegt. Beispielsweise kann die Generatorgrundfrequenz gleich der Parallelschwingkreis-Resonanzfrequenz gewählt werden, z.B. 350 kHz. Die Resonanzfrequenz des Serienschwingkreises beträgt dann z.B. das 1,1-fache der Parallelschwingkreis-Resonanzfrequenz, nämlich 385 kHz (vgl. Fig.2). Die Generatorkurzschlussfrequenz beträgt 500 kHz und liegt somit deutlich über der Serienkreisresonanzfrequenz. Die strichpunktierten Linien zeigen die Impedanz des Paralleschwingkreises 1,2 unter verschiedenen Lastzuständen. Die durchgestrichene Linie ist die Hüllkurve. Im Leerlauf des elektrochirurgischen HF-Generators (Lastwiderstand R_Last sehr groß, angedeutet als R_Last>>) hat die Impedanz ein Maximum bei der Grundfrequenz von 350kHz. Bei zunehmender Belastung d.h. abnehmendem Lastwiderstand (R_Last<) verschiebt sich das Maximum zu höheren Frequenzen, z.B. zu 385kHz. Die Impedanz des Serienschwingkreises 3,4 hat bei dieser Frequenz (385 kHz) ein Minimum. Deshalb sind bei einer Arbeitsfrequenz von 385 kHz Spannung und Strom in Phase, d.h. der Wirkungsgrad ist unter der dieser Frequenz entsprechenden Last optimiert. Bei einem Kurzschluss (R_Last<<) liegt das Maximum der Paralleschwingkreisimpedanz bei der Kurzschlussfrequenz von 500kHz.

### Bezugszeichenliste

- 1: Kapazität /Kondensator
- 2: Induktivität / Spule
- 3: Induktivität / Spule
- 4: Auskoppelkondensator
- 5: Schalter
- 6: Kondensator
- 7: Widerstand
- 8: Phasenschieber
- 9: Pulsformerstufe
- 10: Treiberstufe
- 11: Bedienteil
- 12: Netzteil
- 13: Netzeingangsstufe

## Patentansprüche

1. Elektrochirurgischer HF-Generator zum Schneiden und/oder Koagulieren von biologischem Gewebe, mit einem Netzgerät (12) und einer Generatorstufe zum Anregen einer HF-Schwingung in einem einen Parallelschwingkreis (1, 2) und einen damit gekoppelten Serienschwingkreis (3, 4) umfassenden Ausgangsfilter, an das zwei elektroden angeschlossen sind,
wobei der Serienschwingkreis (3, 4) eine Resonanzfrequenz hat, die über der Resonanzfrequenz des Parallelschwingkreises (1, 2) liegt, **dadurch gekennzeichnet, dass** die Resonanzfrequenz des Serienschwingkreises (3, 4) kleiner ist, als die sich bei einem Kurzschluss der Elektroden einstellende Frequenz.

2. Elektrochirurgischer HF-Generator nach Anspruch 1,
**dadurch gekennzeichnet, dass**
die Resonanzfrequenz des Serienschwingkreises (3, 4) zwischen 5% und 25%, vorzugsweise zwischen 7,5% und 12,5%, über der Resonanzfrequenz des Parallelschwingkreises (1, 2) liegt.

3. Elektrochirurgischer HF-Generator nach Anspruch 1,
**dadurch gekennzeichnet, dass**
die Resonanzfrequenz des Serienschwingkreises (3, 4) kleiner ist als das 0,9-fache, vorzugsweise kleiner ist als das 0,8 fache, der sich bei einem Kurzschluss der Elektroden einstellenden Frequenz des HF-Signals.

4. Elektrochirurgischer HF-Generator nach einem der vorhergehenden Ansprüche,
**dadurch gekennzeichnet, dass**
der Parallelschwingkreis (1, 2) über einen Übertrager mit dem Serienschwingkreis (3, 4) gekoppelt ist.

5. Elektrochirurgischer HF-Generator nach Anspruch 4,
**dadurch gekennzeichnet, dass**
der Parallelschwingkreis (1, 2) eine Induktivität hat (2), die Teil des Übertragers ist.

6. Elektrochirurgischer HF-Generator nach Anspruch 4 oder 5,
**dadurch gekennzeichnet, dass**
der Serienschwingkreis (3, 4) beidseits der Sekundärwicklung des Übertragers je eine Induktivität (3) hat.

7. Elektrochirurgischer HF-Generator nach Anspruch 6,
**dadurch gekennzeichnet, dass**
jeder der beiden Induktivitäten (3) eine Kapazität (4) nachgeschaltet ist, die jeweils mit einer Elektrode verbunden sind.

8. Elektrochirurgischer HF-Generator nach einem der vorstehenden Ansprüche,
**dadurch gekennzeichnet, dass**
die Generatorstufe eine Schaltstufe umfasst.

9. Elektrochirurgischer HF-Generator nach einem der vorstehenden Ansprüche,
**dadurch gekennzeichnet, dass**
die Generatorstufe einen Steuereingang hat, der über einen Kondensator (6) an den Parallelschwingkreis (1, 2) gekoppelt ist.

10. Elektrochirurgischer HF-Generator nach Anspruch 9,
**dadurch gekennzeichnet, dass**
zur Erzeugung eines auf Frequenz des HF-Generators synchronisierten Ansteuersignals ein Phasenschieber (8) mit einer nachgeschalteten Pulsformerstufe (9) an den Steuereingang angeschlossen ist.

11. Elektrochirurgischer HF-Generator nach Anspruch 10,
**dadurch gekennzeichnet, dass**
die Ausgangsignale der Pulsformerstufe (9) über ein Bedienteil (11) einer Treiberstufe (10) zugeführt werden.

## Claims

1. Electrosurgical RF generator for cutting and/or coagulating biological tissue, with a power supply unit (12) and a generator stage for exciting an RF oscillation in an output filter, comprising a rejector circuit (1, 2) and an acceptor circuit (3, 4) coupled thereto, to which output filter two electrodes are connected,
wherein
the acceptor circuit (3, 4) has a resonant frequency that lies above the resonant frequency of the rejector circuit (1, 2),
**characterized in that**
the resonant frequency of the acceptor circuit (3, 4) is less than the frequency setting-in in the case of a short-circuit of the electrodes.

2. Electrosurgical RF generator according to Claim 1,
**characterized in that**
the resonant frequency of the acceptor circuit (3, 4) lies between 5% and 25%, preferably between 7.5% and 12.5%, above the resonant frequency of the rejector circuit (1, 2).

3. Electrosurgical RF generator according to Claim 1,
**characterized in that**
the resonant frequency of the acceptor circuit (3, 4) is less than 0.9 times, preferably less than 0.8 times, the frequency of the RF signal setting-in in the case of a short-circuit of the electrodes.

4. Electrosurgical RF generator according to one of the preceding claims,
**characterized in that**
the rejector circuit (1, 2) is coupled to the acceptor circuit (3, 4) by way of a transformer.

5. Electrosurgical RF generator according to Claim 4,
**characterized in that**
the rejector circuit (1, 2) has (2) an inductor, which is part of the transformer.

6. Electrosurgical RF generator according to Claim 4 or 5,
**characterized in that**
the acceptor circuit (3, 4) respectively has an inductor (3) on each of the two sides of the secondary winding of the transformer.

7. Electrosurgical RF generator according to Claim 6,
**characterized in that**
a capacitor (4) is connected downstream of each one of the two inductors (3), which capacitors are each connected to an electrode.

8. Electrosurgical RF generator according to one of the preceding claims,
**characterized in that**
the generator stage comprises a switching stage.

9. Electrosurgical RF generator according to one of the preceding claims,
**characterized in that**
the generator stage has a control input, which is coupled to the rejector circuit (1, 2) by way of a capacitor (6).

10. Electrosurgical RF generator according to Claim 9,
**characterized in that**
a phase shifter (8) with a downstream pulse forming stage (9) is connected to the control input for generating an actuation signal synchronized to the frequency of the RF generator.

11. Electrosurgical RF generator according to Claim 10,
**characterized in that**
the output signals from the pulse forming stage (9) are fed to a driver stage (10) by way of an operating part (11).

## Revendications

1. Générateur HF électro-chirurgical destiné à inciser et/ou coaguler des tissus biologiques, comprenant une unité d'alimentation électrique (12) et un étage générateur destiné à exciter une oscillation HF dans un circuit oscillant parallèle (1, 2) et dans un circuit oscillant série couplé à celui-ci (3, 4), comprenant un filtre de sortie auquel deux électrodes sont connectées, dans lequel
le circuit oscillant série (3, 4) présente une fréquence de résonance qui se situe au-dessus de la fréquence de résonance du circuit oscillant parallèle (1, 2),
**caractérisé en ce que** la fréquence de résonance du circuit oscillant série (3, 4) est inférieure à la fréquence apparaissant lors d'un court-circuit des électrodes.

2. Générateur HF électro-chirurgical selon la revendication 1, **caractérisé en ce que** la fréquence de résonance du circuit oscillant série (3, 4) se situe entre 5 % et 25 %, et de préférence entre 7,5 % et 12,5 %, au-dessus de la fréquence de résonance du circuit oscillant parallèle (1, 2).

3. Générateur HF électro-chirurgical selon la revendication 1, **caractérisé en ce que** la fréquence de résonance du circuit oscillant série (3, 4) est inférieure à 0,9 fois, et de préférence est inférieure à 0,8 fois la fréquence du signal HF qui apparaît lors d'un court-circuit des électrodes.

4. Générateur HF électro-chirurgical selon l'une quelconque des revendications précédentes, **caractérisé en ce que** le circuit oscillant parallèle (1, 2) est couplé au circuit oscillant série (3, 4) par l'intermédiaire d'un transformateur d'isolation.

5. Générateur HF électro-chirurgical selon la revendication 4, **caractérisé en ce que** le circuit oscillant parallèle (1, 2) comporte une inductance (2) qui fait partie du transformateur d'isolation.

6. Générateur HF électro-chirurgical selon la revendication 4 ou 5, **caractérisé en ce que** le circuit oscillant série (3, 4) présente une inductance (3) de chaque côté de l'enroulement secondaire du transformateur d'isolation.

7. Générateur HF électro-chirurgical selon la revendication 6, **caractérisé en ce que** chacune des deux inductances (3) est connectée en aval d'une capacité (4) qui est respectivement connectée à une électrode.

8. Générateur HF électro-chirurgical selon l'une quelconque des revendications précédentes, **caractérisé en ce que** l'étage générateur comprend un étage de commutation.

9. Générateur HF électro-chirurgical selon l'une quelconque des revendications précédentes, **caractérisé en ce que** l'étage générateur comprend une entrée de commande qui est couplée par l'intermédiaire d'un condensateur (6) au circuit oscillant parallèle (1, 2).

10. Générateur HF électro-chirurgical selon la revendication 9, **caractérisé en ce que**, pour générer un signal de commande synchronisé sur la fréquence du générateur HF, un déphaseur (8) comprenant un étage de formation d'impulsion (9) connecté en aval est relié à l'entrée de commande.

11. Générateur HF électro-chirurgical selon la revendication 10, **caractérisé en ce que** les signaux de sortie de l'étage de formation d'impulsion (9) sont acheminés à un étage d'attaque (10) par l'intermédiaire d'un organe de commande (11).
